# NEUE EUROPÄISCHE PATENTSCHRIFT

(11) **EP 2 662 101 B2**
(45) Veröffentlichungstag und Bekanntmachung des Hinweises auf die Entscheidung über den Einspruch: **30.05.2018**
(45) Hinweis auf die Patenterteilung: 07.01.2015
(21) Anmeldenummer: 12167382.6
(22) Anmeldetag: 09.05.2012
(51) Int. Cl.: A61M 1/36

(54) **Verfahren zur Vorfüllung eines Hämodialysegerätes**
Method for priming a haemodialysis device
Procédé de préremplissage d'un appareil d'hémodialyse

(43) Veröffentlichungstag der Anmeldung: 13.11.2013
(73) Patentinhaber: D_MED Consulting AG, 47800 Krefeld (DE)
(72) Erfinder: Biermann, Frank, 22455 Hamburg (DE); Breuch, Gerd, 53844 Troisdorf (DE); Yanagimoto, Yoji, 3080 Tervuren (Moorsel) (BE)
(74) Vertreter: Patentanwälte ter Smitten Eberlein-Van Hoof Rütten Partnerschaftsgesellschaft mbB

(56) Entgegenhaltungen:
- EP-A1- 0 834 329
- EP-A1- 1 457 218
- EP-A1- 1 892 000
- EP-A1- 2 133 107
- EP-A1- 2 218 470
- EP-A2- 0 560 368
- EP-B1- 1 110 566
- EP-B1- 1 327 457
- WO-A1-01/08723
- WO-A1-92/02264
- WO-A1-96/40320
- WO-A1-2008/125893
- WO-A1-2011/081740
- WO-A2-2003/082144
- DE-A1- 3 442 744
- DE-A1-102006 022 122
- DE-B4- 19 655 230
- DE-C1- 10 011 208
- DE-T2- 69 417 608
- DE-T2- 69 428 220
- DE-U1- 9 422 431
- JP-A- 2003 180 823
- JP-A- 2005 218 709
- US-A- 4 324 662
- US-A- 5 863 421
- US-A1- 2004 243 050
- US-A1- 2008 262 405
- US-B1- 6 187 198
- US-B2- 8 114 276
- Artis Operator's Manual von Gambro aus 11/2009, Seiten 1 bis 1092
- Fresenius Medical Care North America Press Release
- HÖRL W.H. ET AL: 'Replacement of Renal Function by Dialysis', Bd. 5TH ED., 2004, KLUWER ACADEMIC PUBLISHERS, DORDRECHT Seiten 325 - 449

## Beschreibung

Die Erfindung bezieht sich auf ein Verfahren zur Vorfüllung eines Hämodialysegerätes.

Ein Hämodialysegerät weist eine Dialysatseite und eine Blutseite auf. Zu der Dialysatseite zählt eine Dialysatquelle, die das Dialysat zur Verfügung stellt, und zwar entweder in Form von durch das Hämodialysegerät mit Additiven versetztem Dialysatwasser oder in Form eines Dialysat-Vorrats. Ferner zählt die Dialysatkammer eines eine Membran aufweisenden Dialysators und eine Dialysatpumpe, die das Dialysat von der Dialysatquelle zu der Dialysatkammer pumpt, zu der Dialysatseite. Schließlich ist auf der Dialysatseite zwischen der Dialysatkammer und einem Abfalltank bzw. einem Abfallablauf eine Abfallpumpe vorgesehen. Zu der Blutseite zählen die Blutkammer des Dialysators, sowie eine venöse Leitung und eine arterielle Leitung, die beide an den beiden Enden der Dialysator- Blutkammer angeschlossen sind.

Ferner ist eine Vorfüllflüssigkeits-Quelle vorgesehen die Vorfüllflüssigkeit für das Vorfüllen der venösen und der arteriellen Leitung zur Verfügung stellt. Die Vorfüllflüssigkeits-Quelle kann von der Dialysatquelle gebildet werden, kann jedoch grundsätzlich auch eine separate Vorfüllflüssigkeits-Quelle sein, beispielsweise ein Beutel mit einer Kochsalzlösung.

Vor dem Anlegen der venösen Leitung und der arteriellen Leitung bzw. der betreffenden Kanülen an den Patienten werden die beiden blutseitigen Leitungen mit Vorfüllflüssigkeit vorgefüllt, wie dies beispielsweise aus DE 196 55 224 B4 bekannt ist. Das Einleiten der Vorfüllflüssigkeit kann durch eine Pumpe unterstützt werden, beispielsweise durch die Dialysatpumpe, die in der Regel als Verdrängerpumpe in Form einer peristaltischen Schlauchpumpe ausgebildet ist. Hierdurch besteht grundsätzlich die Gefahr, dass beim Vorfüllen der Fluiddruck in der venösen Leitung und/oder der arteriellen Leitung so stark ansteigt, beispielsweise verursacht durch einen Leitungsknick, dass unzulässige Überdrücke auftreten oder die betreffende Leitung beschädigt werden kann, und hierdurch, beispielsweise undicht werden kann. Falls die Undichtigkeit zunächst unbemerkt bleibt, kann sich der Aufwand für die Vorbereitung der Dialyse erheblich erhöhen.

Aus WO 2008/125893 ist ein Vorfüll-Verfahren bekannt, bei dem das Vorfüllen mit einem konstanten Fülldruck, also druckgeregelt erfolgt.

Aufgabe der Erfindung ist es demgegenüber, ein verbessertes Verfahren zur Vorfüllung eines Hämodialysegerätes zu schaffen.

Diese Aufgabe wird erfindungsgemäß durch ein Verfahren zur Verfügung eines Hämodialysegerätes mit den Merkmalen des Anspruchs 1 gelöst.

Auf der Dialysatseite ist mindestens ein Drucksensor vorgesehen. Während des Vorfüllens der arteriellen und der venösen Leitung mit Vorfüllflüssigkeit aus der Vorfüllflüssigkeits-Quelle wird der Fluiddruck ständig durch den Drucksensor überprüft, wobei das Vorfüllen gestoppt wird, sobald und solange der von dem betreffenden Drucksensor gemessene blutseitige Fluiddruck einen festgesetzten Grenzdruck unter- bzw. überschreitet. Der festgesetzte Grenzdruck ist der Höhe nach so gewählt, dass die Gefahr einer Beschädigung der blutseitigen Leitungen ausgeschlossen werden kann, solange der blutseitige Fluiddruck den festgelegten Grenzdruck nicht unter- bzw. übersteigt.

Die Gefahr eines zu hohen blutseitigen Fluiddrucks kann sich beispielsweise dadurch ergeben, dass die in der Regel flexibel ausgebildeten blutseitigen Leitungen geknickt werden, so dass sich der Flusswiderstand an der betreffenden Knickstelle erhöht oder die Leitung an der Knickstelle vollständig blockiert wird. Dies kann während des Vorfüllens durch eine quasi-kontinuierliche Überwachung des blutseitigen Fluiddrucks in der arteriellen und/oder der venösen Leitung detektiert werden. Abhängig von der fluidischen Position des betreffenden Drucksensors im Verhältnis zu einer Pumpe steigt oder sinkt der von dem Drucksensor detektierte Fluiddruck bei einem erhöhten Flusswiderstand in den blutseitigen Leitungen. Im Falle einer Unter- bzw. Überschreitung des Grenzdrucks wird zum Einen das Vorfüllen gestoppt, und kann zum Anderen ein entsprechendes Warnsignal ausgegeben werden.

Vorzugsweise wird die Vorfüllflüssigkeits- Quelle von einer Dialysatquelle gebildet, und wird beim Vorfüllen die Vorfüllflüssigkeit von der Dialysatpumpe durch die Dialysator-Membran hindurch in die Blutkammer, und von dort in die arterielle und die venöse Leitung gepumpt. Die Vorfüllflüssigkeit wird also nicht aus einem separaten Vorfüllflüssigkeits-Tank direkt in die blutseitigen Leitungen eingeleitet, sondern indirekt durch den Dialysator von der Dialysatkammer aus in die Blutkammer eingeleitet, von wo aus die Vorfüllflüssigkeit in die beiden blutseitigen Leitungen fließt. Dieses Vorgehen bietet den Vorteil, dass ein separater Vorfüllflüssigkeits-Vorrat nicht erforderlich ist.

Die Pumpen auf der Dialysatseite, also die Dialysatpumpe und die Abfallpumpe, sind als sehr genau und fehlerfrei arbeitende Verdrängerpumpen ausgebildet, beispielsweise als Membranpumpen Kolbenpumpen bzw. Bilanzkammerpumpe. Dieser Umstand hat zur Folge, dass bei einem erhöhten Strömungswiderstand in einer der blutseitigen Leitungen, sich der Fluiddruck in der betreffenden blutseitigen Leitung dramatisch erhöhen kann. Durch eine ständige und zeitlich engmaschige Kontrolle des Fluiddrucks in den blutseitigen Leitungen kann ein derartiger dramatischer Druckanstieg sofort bemerkt werden, und die für den Füllflüssigkeitsstrom durch die Dialysator-Membran verantwortliche Pumpe abgeschaltet werden. Die Gefahr einer unbemerkten Beschädigung und von Lufteinschlüsse in bzw. an den blutseitigen Leitungen kann auf diese Weise erheblich reduziert werden.

Gemäß einer bevorzugten Ausgestaltung ist die Dialysatpumpe zwangsweise mit der Abfallpumpe zu einer Bilanzierpumpe gekoppelt, beispielsweise mechanisch gekoppelt. Hierdurch wird mechanisch sichergestellt, dass die Dialysatpumpe jeweils exakt dieselbe Flüssigkeitsmenge in Richtung Dialysator pumpt, wie die Abfallpumpe aus Richtung Dialysator abpumpt. Ferner ist eine separate Ultrafiltrationspumpe vorgesehen, die fluidisch parallel zu der Abfallpumpe angeordnet ist. Die Ultrafiltrationspumpe wird beim Vorfüllen der blutseitigen Leitungen mit Vorfüllflüssigkeit im Gegenstrom betrieben, d.h., sie pumpt einen Teil der von der Abfallpumpe stromabwärts gepumpten Flüssigkeit über einen Bypass zurück zur Einlassseite der Abfallpumpe. Hierbei liegt die Pumprate der Ultrafiltrationspumpe stets unter der Pumprate der Abfallpumpe, damit nicht bereits verbrauchtes Dialysat rückwärts in den Dialysator bzw. in die blutseitigen Leitungen gepumpt wird.

Vorzugsweise pumpt die in der arteriellen Leitung oder der venösen Leitung angeordnete Blutpumpe in Richtung eines freien Leitungsendes mit einer Pumprate, die niedriger ist als die Vorfüllungs-Pumprate, mit der die Vorfüllflüssigkeit durch die Dialysator-Membran von der Dialysatkammer zu der Blutkammer hindurchtritt. Die Pumprate der Blutpumpe kann beispielsweise derart gewählt und eingestellt sein, dass sich die arterielle Leitung und die venöse Leitung gleichmäßig mit Vorfüllflüssigkeit füllen bzw. ungefähr zum selben Zeitpunkt vollständig mit Vorfüllflüssigkeit gefüllt sind.

Gemäß einer bevorzugten Ausgestaltung wird vor dem Starten des Vorfüllens das venöse Leitungsende mit der arteriellen Leitung oder das arterielle Leitungsende mit der venösen Leitung direkt verbunden. Besonders bevorzugt ist im Verlauf der venösen Leitung eine Luftfalle angeordnet, die eine Vorfüllungs-Kupplung zum Ankuppeln des arteriellen Leitungsendes während des Vorfüllens aufweist. Auf diese Weise kann sichergestellt werden, dass während des Vorfüllens beide blutseitigen Leitungen an die Luftfalle angeschlossen sind, so dass Luft aus beiden blutseitigen Leitungen in der Luftfalle entfernt wird.

Vorzugsweise ist im Verlauf der venösen Leitung zwischen der Luftfalle und der venösen Kanüle eine aktuierbare venöse Leitungsklemme vorgesehen. Die arterielle Leitung ist mit ihrem Leitungsende zwischen der venösen Leitungsklemme und der Luftfalle an die venöse Leitung bzw. direkt an die Luftfalle angeschlossen. Während der Vorfüllung ist eine Entlüftungsphase mit den folgenden Verfahrensschritten vorgesehen:
Stoppen des Vorfüllens,
Schließen der venösen Leitungsklemme, und

Betrieb der Blutpumpe zum Pumpen der Vorfüllflüssigkeit von der Luftfalle durch die venöse Leitung zu der Dialysator-Blutkammer.

Zunächst wird das Vorfüllen der Blutseite mit Vorfüllflüssigkeit gestoppt, beispielsweise durch Anhalten der Ultrafiltrationspumpe. Es tritt keine Vorfüllflüssigkeit mehr von der Dialysatseite zur Blutseite über. Durch das anschließende Schließen der venösen Leitungsklemme wird die Vorfüllflüssigkeit in den blutseitigen Leitungen durch die Blutpumpe nur noch im Kreis gepumpt bzw. gefördert. Auf diese Weise wird das gesamte Vorfüllflüssigkeits-Volumen so oft, wie gewünscht, im blutseitigen Leitungskreis im Kreis gepumpt, so dass das gesamte Vorfüllflüssigkeits-Volumen entsprechend häufig die Luftfalle passiert, so dass in der Luftfalle die gesamte Luft aus den blutseitigen Leitungen entfernt werden kann.

Vorzugsweise ist zwischen der Dialysator-Blutkammer und der arteriellen Kanüle eine arterielle Leitungsklemme vorgesehen. Während der Entlüftungsphase wird zwischen dem Schließen und dem Öffnen der venösen Leitungsklemme die arterielle Leitungsklemme geschlossen und geöffnet. Während die Vorfüllflüssigkeit in den blutseitigen Leitungen durch die Blutpumpe im Kreis gefördert wird, wird die venöse Leitungsklemme geschlossen und dann wieder geöffnet. Hierdurch wird ein Druckstoß in die Vorfüllflüssigkeits-Säule in den blutseitigen Leitungen induziert, durch den festsitzende Luftbläschen losgerissen und gelöst, und von der Vorfüllflüssigkeit mitgerissen werden können, die auf diese Weise schließlich in die Luftfalle gepumpt werden. Das Schließen und Öffnen der arteriellen Leitungsklemme kann auch mehrmals und in einer Vielzahl kurzer Intervalle erfolgen, so dass in kurzer Zeit viele entsprechende Druckstöße induziert werden können.

Alternativ oder ergänzend kann die Pumprate der Blutpumpe während der Entlüftungsphase variiert werden, beispielsweise in einem bestimmten Rhythmus auf das Doppelte erhöht werden.

Vorzugsweise bleibt die Dialysator-Blutkammer bzw. der Dialysator während der gesamten Vorfüllung, und insbesondere auch während der Entlüftungsphase, unverändert vertikal angeordnet bzw. ausgerichtet, so dass die venöse Leitung unten an die Blutkammer und die arterielle Leitung oben an die Blutkammer angeschlossen ist. Ein Wenden des Dialysators, beispielsweise zur Entlüftung, ist nicht mehr erforderlich. Hierdurch wird die Handhabung bei der Vorfüllung der blutseitigen Leitungen erheblich vereinfacht und eine Fehlerquelle völlig vermieden.

Vorzugsweise weist die Luftfalle ein Tauchrohr mit einer unterhalb eines Flüssigkeitspegels liegenden Rohröffnung auf, wobei das Tauchrohr über einem Abschnitt der venösen Leitung direkt mit der Blutkammer des Dialysators verbunden ist. Dadurch, dass die Rohröffnung unterhalb des Flüssigkeitspegels in der Luftfalle liegt, kann die Luftfalle in beiden Strömungsrichtungen betrieben werden, also auch im Gegenstrom, ohne dass hierdurch Luft aus dem Luftvolumen oberhalb des Flüssigkeitspegels in die blutseitigen Leitungen gelangen kann. Insbesondere während der Entlüftungsphase wird die Luftfalle im Gegenstrom betrieben.

Im Folgenden wird unter Bezugnahme auf die Zeichnungen ein Ausführungsbeispiel des erfindungsgemäßen Verfahrens näher erläutert.

Es zeigen:
Figur 1 eine schematische Darstellung eines Hämodialysegerätes zur Darstellung eines Verfahrens zur Vorfüllung des Hämodialysegerätes, während des Vorfüllens der arteriellen und der blutseitigen Leitungen mit Vorfüllflüssigkeit, und
Figur 2 die schematische Darstellung des Hämodialysegerätes der Figur 1 während einer Entlüftungsphase.

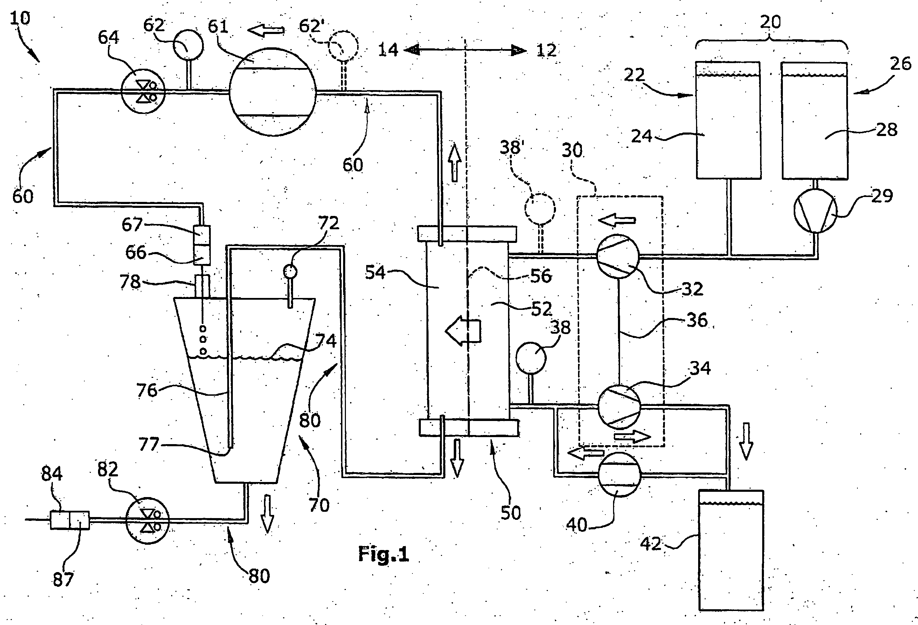

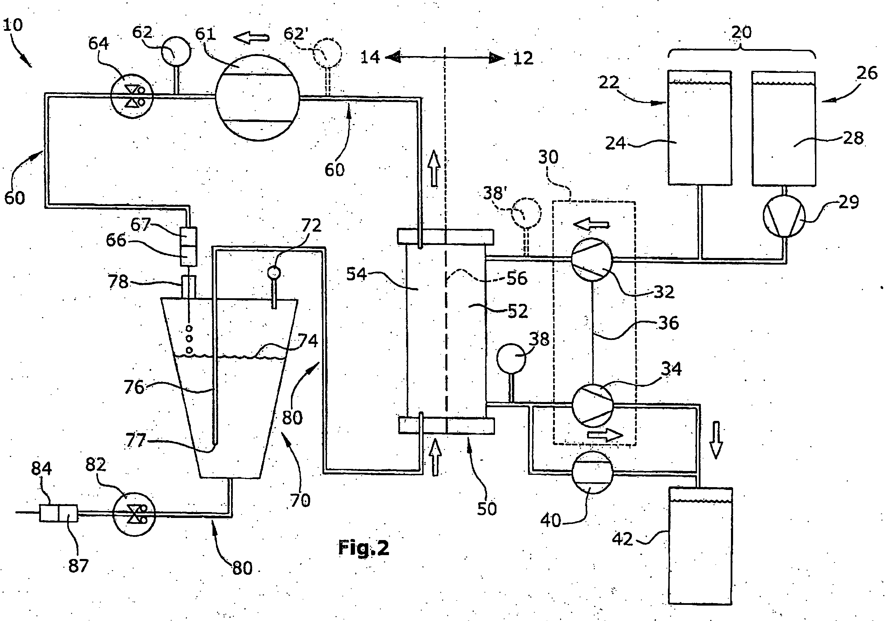

In den Figuren 1 und 2 ist schematisch ein Hämodialysegerät 10 dargestellt, das funktional in eine Dialysatseite 12 und eine Blutseite 14 unterteilt werden kann. Die Grenze zwischen der Dialysatseite 12 und der Blutseite 14 wird von einer Dialyse-Membran 56 in einem Dialysator 50 gebildet. Die Dialyse-Membran 56 trennt in dem Dialysator 50 eine Dialysatkammer 52 von einer Blutkammer 54.

Die Dialysatseite 12 weist eine Vorfüllflüssigkeits-Quelle 20 auf, die eine Vorfüllflüssigkeit zur Verfügung stellt. Vorliegend ist die Vorfüllflüssigkeits-Quelle 20 auch eine Dialysatquelle, die Dialysat für die Dialyse zur Verfügung stellt. Die Vorfüllflüssigkeits-Quelle 20 wird von einem Dialysewasser-Tank 22 mit Dialysewasser 24 und einem Additiv-Tank 26 mit einem Dialysat-Additiv 28 gebildet. Dem Additiv-Tank 26 ist eine Additiv-Pumpe 29 nachgeordnet, die das Additiv 28 bei Bedarf wohldosiert dem Dialysewasser-Strom zuführt. Es können auch mehrere Additiv-Tanks vorgesehen sein, wobei als Additiv insbesondere Elektrolyte und Puffersubstanzen vorgesehen sind. Das Dialysewasser kann, alternativ zu dem Dialysewasser-Tank 22, auch von einer (nicht dargestellten) Wasseraufbereitungs-vorrichtung zur Verfügung gestellt werden, in der aus Trinkwasser aus dem öffentlichen Leitungsnetz das Dialysewasser hergestellt wird.

Das Dialysat bzw. die identische Vorfüllflüssigkeit aus der Vorfüllflüssigkeits-Quelle 20 wird durch eine Dialysatpumpe 32 in die Dialysatkammer 52 des Dialysators 50 gepumpt. Von der Dialysatkammer 52 aus verläuft eine Leitung zu einer Abfallpumpe 34, die das Dialysat bzw. die Vorfüllflüssigkeit aus der Dialysatkammer 52 in einen Abfalltank 42 pumpt, in dem das verbrauchte Dialysat bzw. die überschüssige Vorfüllflüssigkeit gespeichert wird. Fluidisch parallel zu der Abfallpumpe 34 ist eine Ultrafiltrationspumpe 40 angeordnet, die in beiden Richtungen betrieben werden kann, also im Gegenstrom und im Parallelstrom zu der Abfallpumpe 34 betrieben werden kann.

Die Dialysatpumpe 32 und die Abfallpumpe 34 sind mechanisch durch eine mechanische Verbindung 36 miteinander verbunden, so dass die Pumpraten der Dialysatpumpe 32 und der Abfallpumpe 34 stets absolut identisch sind. Auf diese Weise bilden die Dialysatpumpe 32 und die Abfallpumpe 34 eine sogenannte Bilanzierpumpe 30. Die mechanische Verbindung 36 kann beispielsweise dadurch realisiert sein, dass die Bilanzierpumpe 30 als Membranpumpe ausgebildet ist, wobei die eine Seite der Pumpenmembran die Pumpkammer der Dialysatpumpe 32 und die andere Seite der Pumpenmembran die Pumpkammer der Abfallpumpe 34 bildet. Wenn die Ultrafiltrationspumpe 40 im Gegenstrom betrieben wird, wird mit exakt der Pumprate der Ultrafiltrationspumpe Vorfüllflüssigkeit von der Blutseite 12 durch die Dialysator-Membran 56 hindurch zur Dialysatseite 14 gepumpt. Zwischen der Dialysatkammer 52 und der Abfallpumpe 34 ist ein Drucksensor 38 angeordnet.

Auf der Blutseite 14 der Dialysator-Membran 56 befindet sich die Blutkammer 54 des Dialysators 50, in die eine arterielle Leitung 60 und eine venöse Leitung 80 münden. Der Dialysator 50 ist an dem Hämodialysegerät 10 senkrecht fixiert, so dass die Strömungsrichtung sowohl in der Blutkammer 54 als auch in der Dialysatkammer 52 senkrecht orientiert ist, und während der Blutwäsche im Gegenstrom verläuft.

Im Verlauf der arteriellen Leitung 60 ist eine Blutpumpe 61 angeordnet, die als peristaltische Schlauchpumpe ausgebildet ist und in beiden Pumprichtungen betrieben werden kann. Die Pumprichtung und Pumprate der Blutpumpe 61 wird von einer nicht dargestellten Gerätesteuerung gesteuert. Im weiteren Verlauf der arteriellen Leitung 60 ist ein arterieller Drucksensor 62 angeordnet, durch den der statische Fluiddruck in der arteriellen Leitung hinter der Blutpumpe 61 detektiert wird. Der arterielle Drucksensor 62' kann auch zwischen der Blutpumpe 61 und dem Dialysator 50 angeordnet sein. Auch der arterielle Drucksensor 62 ist mit der Gerätesteuerung verbunden. Hinter dem arteriellen Drucksensor 62 ist eine arterielle Schlauchklemme 64 angeordnet, die die als flexibler Schlauch ausgebildete arterielle Leitung 60 schließen oder öffnen kann. Am Leitungsende 67 der arteriellen Leitung 60 wird zu einem gegebenen Zeitpunkt eine arterielle Kanüle 66 appliziert, die eine Nadel aufweist, mit der die arterielle Leitung 60 an ein Blutgefäß eines Patienten appliziert werden kann, aus dem die Blutpumpe 61 zur Durchführung der eigentlichen Blutreinigung Patientenblut in die arterielle Leitung 60 absaugt.

Im Verlauf der venösen Leitung 80 ist eine konische Luftfalle 70 angeordnet, die ein mittiges und steifes Tauchrohr 76 mit einer nach unten öffnenden Rohröffnung 77 aufweist. Die Rohröffnung 77 liegt weit unter-halb eines Flüssigkeitspegels 74, dessen Höhe über einen separaten obenliegenden Gasanschluss (nicht dargestellt) und eine daran angeschlossene Pegelregelpumpe (nicht dargestellt) auf einen Sollpegel geregelt wird. Auf diese Weise wird sichergestellt, dass die Rohröffnung 77 stets unterhalb des Flüssigkeitspegels 74 liegt. Dadurch wird sichergestellt, dass die Luftfalle 70 grundsätzlich in beiden Richtungen betrieben werden kann.

An der Luftfalle 70 ist ferner ein venöser Drucksensor 72 angeordnet, der den Fluiddruck in der venösen Leitung 80 detektiert. Schließlich weist die Luftfalle 70 noch eine Vorfüllungs-Kupplung 78 auf, an die das arterielle Leitungsende 67 oder ggf. die arterielle Kanüle 66 angeschlossen werden kann. Während der gesamten Vorfüllung des Hämodialysegeräts 10 bleibt das arterielle Leitungsende 67 bzw. die arterielle Kanüle 66 an die Vorfüllungs-Kupplung 78 angeschlossen. Erst zur Durchführung der eigentlichen Blutreinigung wird das arterielle Leitungsende 67 bzw. die arterielle Kanüle 66 von der Vorfüllungs-Kupplung 78 abgenommen und über die arterielle Kanüle 66 an ein arterielles Blutgefäß des Patienten angeschlossen.

Im weiteren Verlauf der als flexibler Schlauch ausgebildeten venösen Leitung 80 ist eine venöse Leitungsklemme 82 vorgesehen, die mit der Gerätesteuerung verbunden ist und ein schaltbares Ventil darstellt, das die venöse Leitung 80 schließt oder öffnet. An dem freien Leitungsende 87 der venösen Leitung 80 ist eine venöse Kanüle 84 vorgesehen, die eine Nadel aufweist, so dass die venöse Kanüle 84 zu Beginn der eigentlichen Blutreinigung an ein entsprechendes venöses Blutgefäß des Patienten angeschlossen werden kann.

Bei Inbetriebnahme des Hämodialysegeräts werden zunächst die als Einmalartikel ausgebildeten blutseitigen Leitungen 60,80 in das Hämodialysegerät 10 appliziert. Anschließend wird das arterielle Leitungsende 67 bzw. die arterielle Kanüle 66 an die Vorfüllungs-Kupplung 78 der Luftfalle 70 angekoppelt. Die Vorfüllung des Hämodialysegerätes 10 kann nun gestartet werden, indem die Bilanzierpumpe 30 eingeschaltet wird. Hierdurch wird die Vorfüllflüssigkeit, die sich aus dem Dialysewasser 24 und dem Dialysat-Additiv 28 zusammensetzt, durch die Dialysatpumpe 32 in die Dialysatkammer 52 des Dialysators 50, und von dort durch die Abfallpumpe 34 in den Abfalltank 42 gepumpt. Die Pumprate der Bilanzierpumpe 30 beträgt beispielsweise 500 ml/min. Sobald die Vorfüllflüssigkeit den Abfalltank 42 erreicht, wird die Ultrafiltrationspumpe 40 im Gegenstrom eingeschaltet, und zwar mit einer Pumprate unterhalb der Bilanzierpumpen-Pumprate, beispielsweise mit 400 ml/min.

Hierdurch tritt die Vorfüllflüssigkeit mit einer Vorfüllungs-Pumprate, die der Ultrafiltrationspumpen-Pumprate entspricht, aus der Dialysatkammer 52 durch die Dialysator-Membran 56 hindurch in die Blutkammer 54. Die Blutpumpe 61 wird mit einer Pumprate nicht größer als die Vorfüllungs-Pumprate betrieben, beispielsweise mit 200 ml/min, so dass jeweils etwa der halbe Vorfüllflüssigkeits-Strom aus der Blutkammer 54 in die arterielle Leitung 60 und in die venöse Leitung 80 fließt. Die beiden Leitungsklemmen 64,82 sind hierbei geöffnet. Dieser Zustand wird als Vorfüllen bezeichnet und ist in der Figur 1 dargestellt.

Während des Vorfüllens bestimmt und kontrolliert die Gerätesteuerung ständig den Fluiddruck in der arteriellen Leitung 60 und in der venösen Leitung 80. Sobald der gemessene Fluiddruck einen festgelegten Grenzdruck überschreitet, wird das Vorfüllen sofort gestoppt, um eine Beschädigung oder ein Platzen der arteriellen Leitung 60 und/oder der venösen Leitung 80 zu verhindern. Eine Überschreitung des festgelegten Grenzdrucks kann insbesondere dann auftreten, wenn eine der blutseitigen Leitungen 60,80 geknickt ist. Der Fluiddruck wird mit einem oder mehreren dialysatseitigen Druckaufnehmern 38; 38' bestimmt.

Zum Abschluss des Vorfüllens oder als Unterbrechung des Vorfüllens ist eine Entlüftungsphase vorgesehen, die in der Figur 2 dargestellt ist. Hierbei wird die Ultrafiltrationspumpe 40 angehalten, so dass keine Vorfüllflüssigkeit durch die Dialysator-Membran 56 hindurchtritt. Die Pumprate der Bilanzierpumpe 30 kann in der Entlüftungsphase auf beispielsweise 100 ml/min reduziert werden. Gleichzeitig mit dem Anhalten der Ultrafiltrationspumpe 40 wird die venöse Leitungsklemme 82 geschlossen, so dass die Vorfüllflüssigkeit nicht mehr von der Luftfalle 70 zu dem venösen Leitungsende 87 bzw. einen daran angeschlossenen Ablaufbeutel oder Ablaufport fließen kann. Die Blutpumpe 61 wird weiterhin in derselben Richtung wie beim Vorfüllen betrieben, so dass die Vorfüllflüssigkeit in einem Kreislauf gepumpt wird, wobei die Vorfüllflüssigkeit am unteren Ende der Dialysator-Blutkammer 54 durch die venöse Leitung 80 in die Dialysator-Blutkammer 54 eintritt und an dem vertikal oberen Ende aus der Blutkammer 54 aus- und in die arterielle Leitung 60 eintritt. Es entsteht in der Blutkammer 54 ein von unten vertikal nach oben verlaufender Vorfüllflüssigkeits-Strom, durch den Luftbläschen in ihrer Auftriebsrichtung mitgerissen werden.

Auf diese Weise werden Luftbläschen aus der Blutkammer 54 entfernt, die schließlich in der Luftfalle 70 aus dem Vorflüssigkeits-Strom endgültig entfernt werden. Während der Entlüftungsphase wird die arterielle Leitungsklemme 64 periodisch geschlossen und geöffnet, um auf diese Weise Druckstöße in den geschlossenen Vorfüllflüssigkeits-Kreislauf zu induzieren. Alternativ oder ergänzend kann auch die Pumprate der Blutpumpe rhytmisch variiert werden, z.B. mit 200 ml/s und 400 ml/s, wodurch Druckstöße in die Leitungen induziert werden. Durch die induzierten Druckstöße wird das Ablösen der Luftbläschen in dem gesamten Kreislauf, und insbesondere in der Blutkammer 54, verbessert. Der Dialysator 50 bleibt während der gesamten Vorfüllung einschließlich der Entlüftungsphase(n) unverändert vertikal angeordnet, und muss nicht um 180° gedreht werden, um ihn zu entlüften. Die venöse Leitung 80 bleibt also stets vertikal unten an die Blutkammer 54 und die arterielle Leitung 60 stets oben an die Blutkammer 54 angeschlossen.

Nach der Entlüftungsphase kann die Vorfüllung des Hämodialysegerätes beendet werden oder aber das Vorfüllen fortgesetzt werden. Bei Beendigung der Vorfüllung wird die arterielle Kanüle 66 von der Kupplung 78 abgekuppelt und an das betreffende Blutgefäß des Patienten appliziert. Auch die applizierte venöse Kanüle 84 kann an das betreffende Blutgefäß des Patienten appliziert, so dass mit der Blutreinigung des Patientenblutes begonnen werden kann.

## Patentansprüche

1. Verfahren zur Vorfüllung eines Hämodialysegerätes (10), wobei das Hämodialysegerät (10) aufweist:
eine Vorfüllflüssigkeits- Quelle (20), die Vorfüllflüssigkeit zur Verfügung stellt,
eine Dialysatseite (12) mit einer Dialysatkammer (52) eines eine Membran (56) aufweisenden Dialysators (50), einer Dialysatpumpe (32), die die Vorfüllflüssigkeit zu der Dialysatkammer (52) pumpt, einer Abfallpumpe (34), die die Vorfüllflüssigkeit von der Dialysatkammer (52) weg pumpt,
eine Blutseite (14) mit einer arteriellen Leitung (60), einer Blutpumpe (61), einer Blutkammer (54) des Dialysators (50), einer venösen Leitung (80),
und einem Drucksensor (38, 38') auf der Dialysatseite (12) mit den Verfahrensschritten:
Vorfüllen der arteriellen und der venösen Leitung (42,44) mit Vorfüllflüssigkeit aus der Vorfüllflüssigkeits-Quelle (16) durch Betrieb der Dialysatpumpe (32), und
während des Vorfüllens: ständige Bestimmung des blutseitigen Fluiddrucks durch den Drucksensor (38,38'), wobei das Vorfüllen gestoppt wird, wenn der von dem Drucksensor (38,38') gemessene Fluiddruck einen festgelegten Grenzdruck überschreitet bzw. unterschreitet.

2. Verfahren zur Vorfüllung eines Hämodialysegerätes (10) Anspruch 1, wobei die Vorfüllflüssigkeits- Quelle (20) von der Dialysatquelle gebildet wird, und beim Vorfüllen die Vorfüllflüssigkeit von der Dialysatpumpe (32) durch die Dialysator- Membran (31) hindurch in die arterielle und die venöse Leitung (60,80) gepumpt wird.

3. Verfahren zur Vorfüllung eines Hämodialysegerätes (10) nach einem der vorangegangenen Ansprüche, wobei die Dialysatpumpe (32) zwangsweise mit der Abfallpumpe (34) zu einer Bilanzpumpe (30) gekoppelt ist, und eine separate Ultrafiltrationspumpe (40) fluidisch parallel zu der Abfallpumpe (34) vorgesehen ist, wobei die Ultrafiltrationspumpe (40) beim Vorfüllen im Gegenstrom zu der Abfallpumpe (34) arbeitet.

4. Verfahren zur Vorfüllung eines Hämodialysegerätes (10) nach einem der vorangegangenen Ansprüche, wobei die Blutpumpe (61) beim Vorfüllen in Richtung des Leitungsendes (67) mit einer Pumprate pumpt, die nicht größer ist als die Vorfüllungs-Pumprate, mit der die Vorfüllflüssigkeit durch die Dialysator-Membran (56) hindurchtritt.

5. Verfahren zur Vorfüllung eines Hämodialysegerätes (10) nach einem der vorangegangenen Ansprüche, wobei vor dem Starten des Vorfüllens das venöse Leitungsende (87) mit der arteriellen Leitung (60) oder das arterielle Leitungsende (67) mit der venösen Leitung (80) verbunden wird.

6. Verfahren zur Vorfüllung eines Hämodialysegerätes (10) nach einem der vorangegangenen Ansprüche, wobei im Verlauf der venösen Leitung (80) eine Luftfalle (70) angeordnet ist, die eine Vorfüllungs-Kupplung (78) zum Ankuppeln des arteriellen Leitungsendes (67) während des Vorfüllens aufweist.

7. Verfahren zur Vorfüllung eines Hämodialysegerätes (10) nach Anspruch 6, wobei in der venösen Leitung (80) zwischen der Luftfalle (70) und dem venösen Leitungsende (87) eine aktuierbare venöse Leitungsklemme (82) vorgesehen ist, und eine Entlüftungsphase mit den folgenden Verfahrensschritten vorgesehen ist:
Stoppen des Vorfüllens,
Schließen der venösen Leitungsklemme (82), und
Betrieb der Blutpumpe (61) zum Pumpen der Vorfüllflüssigkeit von der Luftfalle (70) zu der Dialysator-Blutkammer (54).

8. Verfahren zur Vorfüllung eines Hämodialysegerätes (10) nach Anspruch 7, wobei während der Entlüftungsphase die Pumprate der Blutpumpe (61) mehrfach um mindestens 30% verändert wird.

9. Verfahren zur Vorfüllung eines Hämodialysegerätes (10) nach Anspruch 7, wobei zwischen der Dialysator- Blutkammer (54) und der arteriellen Kanüle (66) eine arterielle Leitungsklemme (64) vorgesehen ist, wobei während der Entlüftungsphase und zwischen dem Schließen und dem Öffnen der venösen Leitungsklemme (82) folgender Verfahrensschritt vorgesehen sind:
Schließen und öffnen der arteriellen Leitungsklemme (64).

10. Verfahren zur Vorfüllung eines Hämodialysegerätes (10) nach einem der vorangegangenen Ansprüche 7 bis 9, wobei die Dialysator-Blutkammer (54) während der gesamten Vorfüllung unverändert vertikal angeordnet bleibt, so dass die venöse Leitung (80) unten an die Blutkammer (54) und die arterielle Leitung (60) oben an die Blutkammer (54) angeschlossen ist.

11. Verfahren zur Vorfüllung eines Hämodialysegerätes (10) nach einem der Ansprüche 6-10, wobei die Luftfalle (70) ein Tauchrohr (76) mit einer unterhalb eines Flüssigkeitspegels (74) liegenden Rohröffnung (77) aufweist, wobei das Tauchrohr (76) mit einem Abschnitt der venösen Leitung (80) direkt mit der Blutkammer (54) des Dialysators (50) verbunden ist.

## Claims

1. A method for pre-filling a hemodialysis apparatus (10), wherein the hemodialysis apparatus (10) comprises:
a pre-filling liquid source (20) supplying pre-filling liquid,
a dialysate side (12) with a dialysate chamber (52) of a dialyzer (50) comprising a membrane (56), a dialysate pump (32) pumping the pre-filling liquid to the dialysate chamber (52), a waste pump (54) pumping the pre-filling liquid away from the dialysate chamber (52),
a blood side (14) with an arterial line (60), a blood pump (61), a blood chamber (54) of the dialyzer (50), a venous line (80),
and a pressure sensor (38, 38') on the dialysate side (12),
the method comprising the following steps:
pre-filling the arterial and venous lines (42, 44) with pre-filling liquid from the pre-filling liquid source (16) by operating the dialysate pump (32), and
during pre-filling: continuous determination of the blood-side fluid pressure by the pressure sensor (38, 38'), wherein the pre-filling process is stopped when the fluid pressure detected by the pressure sensor (38, 38') exceeds or falls below a defined limit pressure.

2. The method for pre-filling a hemodialysis apparatus (10) of claim 1, wherein the pre-filling liquid source (20) is formed by the dialysate source, and, during pre-filling, the dialysate pump (32) pumps the pre-filling liquid through the dialyzer membrane (31) into the arterial and venous lines (60, 80).

3. The method for pre-filling a hemodialysis apparatus (10) of one of the preceding claims, wherein the dialysate pump (32) is forcedly coupled with the waste pump (34) to form a balancing pump (30), and a separate ultra-filtration pump (40) is provided fluidically parallel to the waste pump (34), wherein, during pre-filling, the ultra-filtration pump (40) operates in reverse flow relative to the waste pump (34).

4. The method for pre-filling a hemodialysis apparatus (10) of one of the preceding claims, wherein, during pre-filling, the blood pump (61) pumps towards the line end (67) at a pump rate no higher that the pre-filling pump rate at which the pre-filling liquid flows through the dialyzer membrane (56).

5. The method for pre-filling a hemodialysis apparatus (10) of one of the preceding claims, wherein, prior to the start of pre-filling, the venous line end (87) is connected to the arterial line (60) or the arterial line end (67) is connected with the venous line (80).

6. The method for pre-filling a hemodialysis apparatus (10) of one of the preceding claims, wherein, at a position along the venous line (80), an air trap (70) is arranged which comprises a pre-filling coupling (78) for the connection of the arterial line end (67) during pre-filling.

7. The method for pre-filling a hemodialysis apparatus (10) of claim 6, wherein an actuable venous line clip (82) is provided in the venous line (80) between the air trap (70) and the venous line end (87), and a venting phase is provided which comprises the following method steps:
stopping the pre-filling,
closing the venous line clip (82), and
operating the blood pump (61) to pump the pre-filling liquid from the air trap (70) through the venous line to the dialyzer blood chamber (54).

8. The method for pre-filling a hemodialysis apparatus (10) of claim 7, wherein, during the venting phase, the pump rate of the blood pump (61) is varied several times by at least 30%.

9. The method for pre-filling a hemodialysis apparatus (10) of claim 7, wherein an arterial line clip (64) is provided between the dialyzer blood chamber (54) and the arterial cannula (66), wherein during the venting phase and between the closing and the opening of the venous line clip (82), the following method step is provided:
closing and opening arterial line clip (64).

10. The method for pre-filling a hemodialysis apparatus (10) of one of the preceding claims 7 to 9, wherein the dialyzer blood chamber (54) remains unchanged it a vertical orientation during the entire pre-filling process, so that the venous line (80) is connected at the bottom of the blood chamber (54) and the arterial line (60) is connected at the top of the blood chamber (54).

11. The method for pre-filling a hemodialysis apparatus (10) of one of claims 6 - 10, wherein the air trap (70) comprises an immersed tube (76) with a tube opening (77) situated below a liquid level (74), wherein the immersed tube (76) is connected directly with the blood chamber (54) of the dialyzer (50) via a section of the venous line (80).

## Revendications

1. Procédé de préremplissage d'un appareil d'hémodialyse (10), ledit appareil d'hémodialyse (10) comprenant:
une source de liquide de préremplissage (20) fournissant du liquide de préremplissage,
un côté dialysat (12) avec une chambre de dialysat (52) d'un dialyseur (50) comprenant une membrane (56), une pompe à dialysat (32) pompant ladite liquide de préremplissage vers la chambre de dialysat (52), une pompe à résidus (34) pompant la liquide de préremplissage loin de ladite chambre de dialysat (52),
un côté sanguin (14) avec une ligne artérielle (60), une pompe sanguine (61), une chambre de sang (54) du dialyseur (50), une ligne veineuse (80),
et un capteur de pression (38, 38') sur le côté de dialysat (12),
le procédé comprenant les étapes de procédé:
par l'opération de la pompe à dialysat (32), préremplir les lignes artérielles et veineuses (42, 44) de liquide de préremplissage venant de la source de liquide de préremplissage (16), et
pendant le préremplissage: détermination continue de la pression fluidique sur le côté sanguin par le capteur de pression (38, 38'), le préremplissage étant arrêté si la pression fluidique mesurée par le capteur de pression (38, 38') dépasse ou sous-dépasse une pression limite fixée.

2. Procédé de préremplissage d'un appareil d'hémodialyse (10) selon la revendication 1, dans lequel la source de liquide de préremplissage (20) est formée par la source de dialysat, et, lors du préremplissage, le liquide de préremplissage est pompé par la pompe à dialysat (32) à travers la membrane (31) du dialyseur dans les lignes (60, 80) artérielle et veineuse.

3. Procédé de préremplissage d'un appareil d'hémodialyse (10) selon l'une quelconque des revendications précédentes, dans lequel la pompe à dialysat (32) est couplée de manière forcée avec la pompe de résidus (34) pour former une pompe d'équilibrage (30), et une pompe d'ultrafiltration (40) séparée est prévue en parallèle, au niveau fluidique, à ladite pompe de résidus (34), ladite pompe d'ultrafiltration (40), lors du préremplissage, opérant à contre-courant par rapport à la pompe de résidus (34).

4. Procédé de préremplissage d'un appareil d'hémodialyse (10) selon l'une quelconque des revendications précédentes, dans lequel, lors du préremplissage, ladite pompe sanguine (61) pompe vers l'extrémité (67) de la ligne avec un débit de pompage ne dépassant pas le débit de pompage de préremplissage avec laquelle le liquide de préremplissage traverse la membrane (56) du dialyseur.

5. Procédé de préremplissage d'un appareil d'hémodialyse (10) selon l'une quelconque des revendications précédentes, dans lequel, avant le commencement du préremplissage, l'extrémité (87) de la ligne veineuse est raccordée à la ligne artérielle (60) ou l'extrémité (67) de la ligne artérielle est raccordée à la ligne veineuse (80).

6. Procédé de préremplissage d'un appareil d'hémodialyse (10) selon l'une quelconque des revendications précédentes, dans lequel un piège d'air (70) est disposé le long de la ligne veineuse (80), le piège d'air comprenant un raccord de préremplissage (78) pour l'accouplement de ladite extrémité (67) de la ligne artérielle pendant le préremplissage.

7. Procédé de préremplissage d'un appareil d'hémodialyse (10) selon la revendication 6, dans lequel une pince actionnable (82) pour la ligne veineuse est disposée entre le piège d'air (70) et l'extrémité (87) de la ligne veineuse, et une phase de ventilation est prévue, ladite phase comprenant les étapes suivantes:
arrêter le préremplissage,
fermer la pince actionnable (82) pour la ligne veineuse, et
opérer la pompe sanguine (61) pour pomper le liquide de préremplissage du piège d'air (70) vers la chambre de sang (54) du dialyseur.

8. Procédé de préremplissage d'un appareil d'hémodialyse (10) selon la revendication 7, dans lequel, pendant la phase de ventilation, le débit de pompage de la pompe sanguine (61) est varié plusieurs fois par au moins 30%.

9. Procédé de préremplissage d'un appareil d'hémodialyse (10) selon la revendication 7, dans lequel une pince (64) pour la ligne artérielle est prévue entre la chambre de sang (54) du dialyseur et la canule artérielle (66), l'étape suivante étant prévue pendant la phase de ventilation et entre la fermeture et l'ouverture de la pince (82) de la ligne veineuse:
fermer et ouvrir la pince (64) de la ligne artérielle.

10. Procédé de préremplissage d'un appareil d'hémodialyse (10) selon l'une quelconque des revendications précédentes 7 à 9, dans lequel la chambre de sang (54) du dialyseur reste orientée verticalement pendant tout le préremplissage, de sorte que la ligne veineuse (80) est raccordée en bas de la chambre de sang (54) et la ligne artérielle (60) est raccordée en haut de la chambre de sang (54).

11. Procédé de préremplissage d'un appareil d'hémodialyse (10) selon l'une quelconque des revendications précédentes 6 à 10, dans lequel le piège d'air (70) comprend un tube plongeur (76) avec une ouverture de tube (77) située au-dessous d'un niveau de liquide (74), ledit tube plongeur (76) étant directement raccordé à ladite chambre de sang (54) du dialyseur (50) par une section de la ligne veineuse (80).
